**Europäisches Patentamt**

(19) **European Patent Office**

**Office européen des brevets**

(11) Numéro de publication: **0 160 583**
**B1**

(12) # FASCICULE DE BREVET EUROPÉEN

(45) Date de publication du fascicule du brevet:
**10.08.88**

(51) Int. Cl.⁴: **A 61 B 6/04,** H 05 G 1/64

(21) Numéro de dépôt: **85400362.1**

(22) Date de dépôt: **26.02.85**

(54) **Procédé de contrôle de positionnement d'un patient par rapport à une installation de radiologie, et installation pour la mise en oeuvre de ce procédé.**

(30) Priorité: **28.02.84 FR 8403040**

(43) Date de publication de la demande:
**06.11.85 Bulletin 85/45**

(45) Mention de la délivrance du brevet:
**10.08.88 Bulletin 88/32**

(84) Etats contractants désignés:
**DE IT NL**

(56) Documents cité:
**EP-A-0 064 623
DE-A-2 655 661
DE-A-3 213 363
DE-B-2 514 329
FR-A-2 345 983
FR-A-2 482 444**

(73) Titulaire: **THOMSON- CGR, 13, square Max-Hymans, F-75015 Paris (FR)**

(72) Inventeur: **Klausz, Rémy, THOMSON- CSF SCPI 173, bld Haussmann, F-75015 Paris (FR)**

(74) Mandataire: **Chaverneff, Vladimir, THOMSON- CSF SCPI 19, avenue de Messine, F-75008 Paris (FR)**

LIBER, STOCKHOLM 1988

## Description

L'invention concerne un procédé de contrôle de positionnement d'un patient par rapport a une installation de radiologie, du type mettant en oeuvre les moyens de formation de l'image radiologique elle-même et permettant un positionnement précis avec une irradiation du patient particulièrement faible. L'invention vise aussi une installation de radiologie comportant les moyens de mise en oeuvre de ce procédé.

Dans une salle de radiologie, le positionnement du patient par rapport au faisceau de rayons X est une opération délicate et importante. Il est a l'évidence souhaitable de bien déterminer la zone d'observation choisie, à la fois pour que la région à examiner occupe la plus grande partie du moyen de visualisation (film, écran de télévision) et pour que le champ d'irradiation ne s'étende pas au-delà de la zone nécessaire. On a procédé souvent en utilisant des repères anatomiques externes du patient et en positionnant un faisceau lumineux entre ces repères, ce faisceau lumineux simulant le faisceau de rayons X. Ceci nécessite de prévoir dans l'installation une source lumineuse susceptible d'émettre un faisceau de mêmes caractéristiques géométriques que le faisceau de rayons X. Dans sa mise en oeuvre, ce processus, est néanmoins peu précis. Il nécessite par ailleurs un opérateur possédant de bonnes connaissances anatmiques pour placer correctement le faisceau lumineux par rapport aux repères anatomiques externes. Ceux-ci sont en outre plus ou moins faciles à prendre en compte suivant la morphologie du patient. Le seul avantage de ce processus réside dans le fait que le patient n'est pas exposé aux rayons X pendant tout le temps nécessaire au positionnement.

Dans les installations où la radioscopie est prévue, il est tentant de réaliser le positionnement en exploitant ce mode de fonctionnement. En revanche, cela introduit une irradiation supplémentaire du patient, non utile au diagnostic proprement dit, pendant tout le temps que l'on déplace la table porte-patient. Or ces déplacements, concernant des masses importantes, se font à vitesse relativement faible.

L'invention permet de bénéficier de toute la précision que permet le mode de positionnement par observation radioscopique, tout en réduisant considérablement la dose supplémentaire de rayons X, non utile au diagnostic.

Dans cet esprit l'invention concerne essentiellement un procédé de contrôle de positionnement d'un patient par rapport à une installation de radiologie laquelle comporte une table porte-patient, un ensemble de mesure comprenant une source de rayons X et un récepteur, des moyens pour controler des déplacements relatifs entre ledit ensemble de mesure et ladite table et un système de visualisation incluant une mémoire d'image recevant dudit récepteur des informations numériques représentatives d'une image radiologique et un moyen d'affichage de cette image, caractérisé en ce qu'il consiste à acquérir et mémoriser une image par mise en oeuvre brève dudit système de visualisation, à reproduire ladite image dans un cadre ou périmètre déterminé dudit système d'affichage et à provoquer un décadrage relatif, représentatif desdits déplacements relatifs, entre ledit cadre ou périmètre et ladite image.

Il est à noter que les sous-ensembles les plus coûteux, qui viennent d'être énumérés ci-dessus, pour permettre la mise en oeuvre du procédé (notamment la mémoire d'image) existent dans de nombreuses installations de radiologie modernes. Le moyen d'affichage sera de préférence un récepteur de télévision. Le cadre ou périmètre précité de ce moyen d'affichage désigne par exemple les limites mêmes de l'écran dans le cas ou c'est l'image qui se décadre par rapport à l'écran. On peut cependant envisager facilement une variante où ce serait un masque généré électroniquement sur l'écran qui viendrait recouvrir l'image, cette dernière restant fixe sur le moyen d'affichage. C'est pourquoi l'invention vise tout processus et tous moyens de décadrage relatif entre le cadre ou périmètre précité et l'image.

L'invention concerne aussi une installation de radiologie du type comportant une table porte-patient, un ensemble de mesure comprenant une source de rayons X et un récepteur, des moyens pour contrôler des déplacements relatifs entre ledit ensemble de mesure et ladite table et un système de visualisation incluant une mémoire d'image recevant dudit récepteur informations numériques représentatives d'une image radiologique et un moyen d'affichage de cette image, caractérisée en ce qu'elle comporte en outre des moyens d'élaboration de signaux représentatifs desdits déplacements relatifs et des moyens de décadrage relatif de ladite image par rapport auxdits moyens d'affichage, pilotés par lesdits moyens d'élaboration de signaux.

Dans le cas d'une table porte-patient mobile, les moyens d'élaboration de signaux précités sont associés à ladite table pour que ces signaux soient représentatifs des déplacements de la table. L'invention vise cependant aussi le cas où l'ensemble comprenant la source de rayons X et le récepteur se déplace par rapport à la table suivant au moins une direction prédéterminée. Dans ce cas, au moins une partie desdits moyens d'élaboration de signaux est associée audit ensemble.

L'invention sera mieux comprise et d'autres avantages de celle-ci apparaîtront mieux à la lumière de la description qui va suivre de plusieurs modes de réalisation d'une installation de radiologie mettant en oeuvre le principe de l'invention, donnée uniquement à titre d'exemple et faite en référence aux dessins annexés dans lesquels:

- la figure 1 est un schéma-bloc d'une installation de radiologie comportant les perfectionnements selon l'invention ;

- la figure 2 illustre le moyen d'affichage de cette installation et la détermination du décadrage ;

- la figure 3 est un schéma-bloc d'une autre installation de radiologie incorporant les perfectionnements selon l'invention.

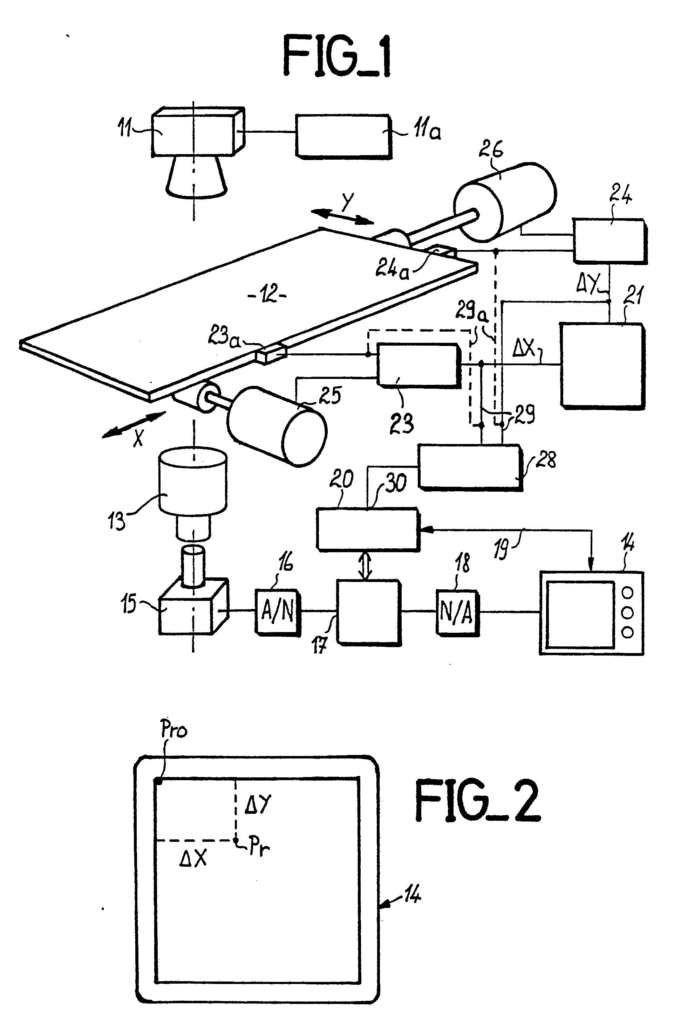

En se reportant plus particulièrement à la figure 1, on a représenté une installation de radiologie moderne mais en grande partie classique, comprenant une source de rayons X, 11 alimentée par un générateur haute tension 11a, une table porte-patient 12 mobile en regard de la source 11, un récepteur constitué ici d'un amplificateur de luminance 13 aligné avec l'axe du faisceau de la source 11 et un téléviseur 14. Dans l'exemple représenté, la table 12 est déplaçable suivant deux directions X, Y et, conformément à une conception classique, ces deux directions sont perpendiculaires entre elles et respectivement parallèles à la longueur et à la largeur du plateau de la table. On pourrait néanmoins adapter le concept de l'invention à n'importe quel type de déplacement de la table.

Une caméra de télévision 15 est couplée à l'amplificateur de luminance pour relever l'image radiologique formée dans ce dernier. Les signaux délivrés par la caméra sont transmis à un convertisseur Analogique-Numérique 16 dont la sortie alimente une mémoire numérique d'image 17. La sortie de la mémoire est couplée au récepteur de télévision 14 par l'intermédiaire d'un convertisseur Numérique-Analogique 18. La mémoire 17 a une capacité suffisante pour stocker sous forme numérique les valeurs de tous les pixels d'une image reproduite sur l'écran du téléviseur 14. Elle est lue cycliquement en permanence sous le contrôle de moyens d'adressage 20 synchronisés avec le téléviseur 14 (liaison 19). La memoire 17 et ses moyens d'adressage 20 sont des sous-ensembles classiques disponibles sur le marché professionnel, ils ne seront donc pas décrits plus en détail. Pour l'exemple spécifiquement décrit, on précise qu'on utilise un équipement développé par la firme Vicom, le mode de fonctionnement de ses moyens d'adressage se prêtant bien à une mise en oeuvre simple et peu coûteuse des principes de l'invention, comme on le verra plus loin. L'installation comporte en outre, de façon classique, un pupitre de saisie 21 des consignes de déplacement de la table 12, selon les directions X, Y. Ce pupitre de saisie convertit les ordres de déplacements de l'opérateur en deux grandeurs électriques $\Delta X$ et $\Delta Y$, servant de tensions de consigne à des circuits d'asservissement 23, 24 pilotant respectivement les moteurs 25, 26 de déplacement de la table 12, selon les directions X, Y. Chaque circuit d'asservissement est aussi connecté à un capteur de position 23a ou 24a respectivement couplé à la table pour délivrer un signal représentatif du déplacement correspondant de celle-ci. Tous ces moyens de commande asservie des moteurs 25 et 26 sont bien connus dans la technique, et existent le plus souvent, indépendemment de l'invention. Cependant, selon l'exemple de la figure 1, on peut dire que le pupitre 21 fait aussi partie des moyens spécifiques à l'invention puisque ses deux sorties délivrant les signaux de consigne $\Delta X$ et $\Delta Y$ sont aussi reliés à un circuit de calcul 28, (liaisons 29) dont la sortie pilote les moyens d'adressage 20. Dans cet exemple, le circuit de calcul 28 et les moyens d'adressage 20 forment des moyens de décadrage de l'image reproduite sur le téléviseur 14. Comme mentionné précédemment, l'agencement des moyens d'adressage 20 se prête particulièrement bien au couplage avec le circuit de calcul 28 et simplifie notablement la conception de ce dernier. En effet, les moyens d'adressage 20 sont conçus pour lire automatiquement les unités de mémorisation de la mémoire 17, dans un ordre correspondant à un balayage ligne par ligne et pixel par pixel sur l'écran du téléviseur 14, connaissant simplement, au début de chaque trame, l'adresse d'un pixel de référence. Bien que ce pixel de référence puisse être quelconque, on supposera pour faciliter la compréhension que la lecture de la mémoire commence à partir de ce pixel, pour la reproduction d'une image donnée, l'exploration de la mémoire se faisant par incrémentation automatique des moyens d'adressage. Par conséquent, lorsqu'on désire une image parfaitement centrée sur l'écran, il suffit d'appliquer à l'entrée de pilotage 30 des moyens d'adressage, un code numérique représentant l'adresse du premier pixel de la première ligne: $Pr_0$.

En revanche, si on considère le référentiel de l'écran du tube cathodique (figure 2), les deux déplacements $\Delta X$ et $\Delta Y$, permettent de déterminer, à partir de $Pr_0$ un nouveau pixel de référence Pr rendant compte d'un décadrage par rapport à l'image d'origine, correspondant aux déplacements de la table. Il suffit donc, d'après ce qui précède, d'appliquer à l'entrée 30, l'adresse du pixel Pr de l'image d'origine (de coordonnées $\Delta X$, $\Delta Y$ dans le référentiel de l'écran), pour réaliser le décadrage voulu. C'est ce que réalise le circuit de calcul 28 à partir des signaux prélevés à la sortie du pupitre 21. La conception du circuit 28 est à la portée de l'homme du métier. Il comporte par exemple des convertisseurs Analogiques-Numériques si les signaux $\Delta X$ et $\Delta Y$ reprsentatifs des déplacements sont des niveaux de tension et deux diviseurs numériques préréglés en fonction de la géométrie du système pour faire correspondre un nombre entier de lignes et de colonnes de l'écran aux déplacements $\Delta X$ et $\Delta Y$, respectivement. Le circuit de calcul comporte aussi des moyens générateurs d'adresse pour convertir en code d'adresse les informations délivrées par les diviseurs et appliquer ce code à l'entrée 30 des moyens d'adressage 20.

Selon une variante également illustrée à la figure 1, les signaux représentatifs des déplacements $\Delta X$, $\Delta Y$ peuvent être prélevés non pas sur les moyens de consigne (c'est-à-dire à la sortie du pupitre 21) mais sur les capteurs de position 23a, 24a. Dans ce cas, les sorties de ces capteurs sont reliées (liaisons 29a en traits interrompus) aux entrées du circuit de calcul 28, les liaisons 29 avec le pupitre 21 étant alors supprimées.

La mise en oeuvre du procédé de l'invention à l'aide du matériel décrit ci-dessus est la suivante. Lorsque le patient a été allongé sur la table 12, l'opérateur procède à l'acquisition d'une image en mode radiocospie, par mise en oeuvre brève de l'ensemble du système de visualisation, c'est-à-dire notamment par une courte émission pulsée de la source de rayons X 11. L'image correspondante est aussitôt mémorisée dans la mémoire 17 et apparaît fixe et parfaitement cadrée au départ, sur l'écran du téléviseur 14, puisque la mémoire 17 est lue cycliquement et continuellement en prenant le pixel $Pr_0$ de l'image enregistrée comme pixel de référence, et cela tant que l'opérateur n'a pas touché aux commandes de déplacement de la table. Lorsque l'opérateur agit sur le pupitre 21 pour déplacer le patient par rapport à l'ensemble source 11-détecteur 13, les signaux correspondants $\triangle X$ et $\triangle Y$ sont traités dans le circuit de calcul 28 pour élaborer l'adresse d'un nouveau pixel de référence, qui apparaît à l'entrée 30. A chaque renouvellement d'image, le décadrage correspondant s'accentue en fonction des déplacements de la table 12. Lorsque le décadrage devient trop important, on procède a nouveau a une mise en oeuvre brève du système de visualisation pour réinscrire les informations numériques correspondant à une nouvelle image non décadrée, dans la mémoire 17 et on poursuit les déplacements de la table en réinitialisant un nouveau décadrage relatif précité. Ces nouveaux "clichés" pris de temps en temps ont l'avantage de faire apparaître de nouveaux détails sur l'écran, qui n'etaient pas visibles sur l'image de départ. Ils peuvent être commandés manuellement sous le contrôle de l'opérateur ou automatiquement. Par exemple, on peut prévoir de renouveler automatiquement l'image lorsque la surface de l'image décadrée sur l'écran tombe en dessous d'un certain pourcentage de la surface totale de l'écran.

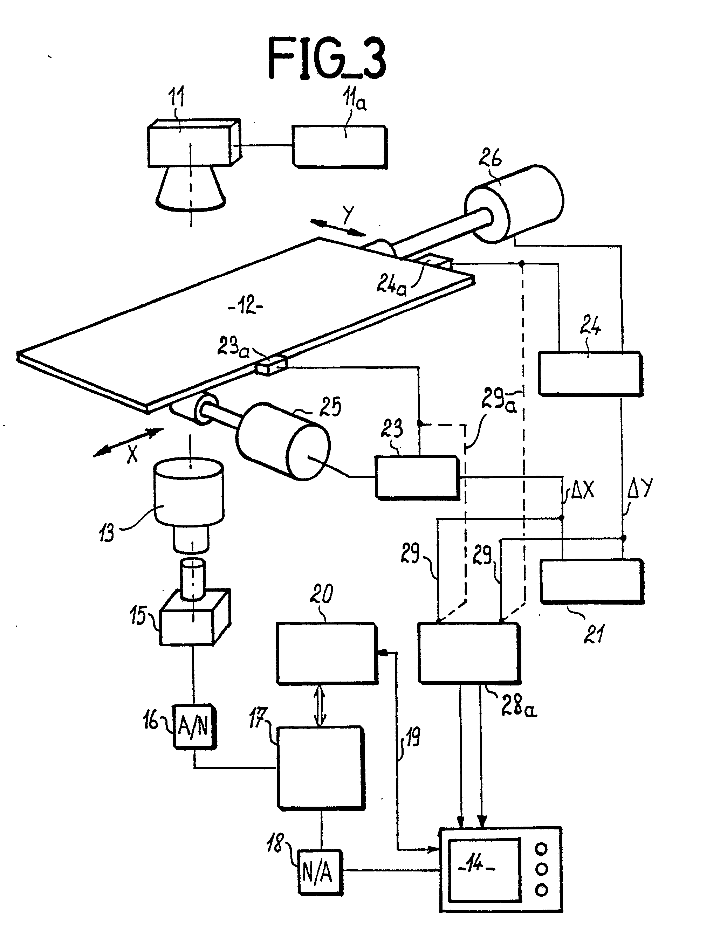

La figure 3 illustre une autre variante plus simple où les moyens de décadrage agissent directement sur le téléviseur 14. Les sous-ensembles de l'installation qui ne changent pas par rapport à la figure 1 portent les mêmes références numériques et ne seront pas décrits à nouveau. Le circuit de calcul 18a ne comporte pas de convertisseur Analogique-Numérique, il peut se resumer en deux amplificateurs de gains prédéterminés, dont les sorties sont connectées aux moyens de déflexion du tube cathodique du téléviseur 14.

De nombreuses autres variantes technologiques sont possibles pour mettre en oeuvre le procédé défini ci-dessus. On peut par exemple convertir les signaux $\triangle X$, $\triangle Y$ en retards proportionnels et faire intervenir ces retards dans la lecture cyclique de la mémoire 17. Le retard correspondant à la grandeur $\triangle Y$ serait par exemple appliqué au début de la lecture de chaque image tandis que le retard correspondant à la grandeur $\triangle X$ serait appliqué au début de la lecture de chaque ligne. C'est dire que l'invention couvre tous les équivalents techniques des moyens mis en oeuvre si ceux-ci le sont dans le cadre des revendications qui suivent.

### Revendications

1. Procédé de contrôle de positionnement d'un patient par rapport à une installation de radiologie, laquelle comporte une table porte-patient (12), un ensemble de mesure comprenant une source de rayons X (11) et un récepteur (13) et des moyens pour contrôler des déplacements relatifs entre ledit ensemble de mesure et ladite table, et un système de visualisation incluant une mémoire d'image (17) recevant dudit récepteur des informations numériques représentatives d'une image radiologique et un moyen d'affichage (14) de cette image, caractérisé en ce qu'il consiste à acquerir et mémoriser une image par mise en oeuvre brève dudit système de visualisation, à reproduire ladite image dans un cadre ou périmètre déterminé dudit moyen d'affichage (14) et à provoquer un décadrage relatif (21, 28, 20), représentatif desdits déplacements relatifs, entre ledit cadre ou périmètre et ladite image.

2. Procédé selon la revendication 1, dans lequel, de façon connue en soi, on lit cycliquement ladite mémoire pour élaborer les signaux appliqués à un tube cathodique de télévision sur lequel ladite image est affichée, caractérisé en ce qu'il consiste à commander des moyens d'adressage (20) de ladite mémoire (17) corrélativement auxdits déplacements relatifs.

3. Procédé selon la revendication 2, caractérisé en ce qu'on élabore des signaux de décadrage représentatifs desdits déplacements relatifs et qu'on les transforme en informations numériques pour commander (28) lesdits moyens d'adressage.

4. Procédé selon la revendication 1, dans lequel, de façon connue en soi, on lit cycliquement ladite mémoire (17) pour élaborer les signaux appliqués à un tube cathodique de télévision sur lequel ladite image est affichée, caractérisé en ce qu'on élabore des signaux de décadrage (28a) représentatifs desdits déplacements relatifs et qu'on les applique à des moyens de déflexion dudit tube cathodique.

5. Procédé selon l'une des revendications précédentes, caractérisé en ce qu'il consiste à acquérir une autre image radiologique par mise en oeuvre brève dudit système de visualisation chaque fois que le décadrage est jugé trop important, à réinscrire les informations numériques correspondant à cette nouvelle image non décadrée dans ladite mémoire d'image (17) et à poursuivre lesdits déplacements relatifs, réinitialisant un nouveau décadrage relatif précité.

6. Installation de radiologie du type comportant une table porte-patient (12), un ensemble de mesure comprenant une source de rayons X (11) et un récepteur (13), des moyens pour contrôler des déplacements relatifs entre ledit ensemble de mesure et ladite table et un système de visualisation incluant une mémoire d'image (17) recevant dudit récepteur des informations numériques représentatives d'une image radiologique et un moyen d'affichage (14) de cette image, caractérisée en ce qu'elle comporte en outre des moyens d'élaboration de signaux (21) représentatifs desdits déplacements relatifs et des moyens de décadrage relatif de ladite image par rapport auxdits moyens d'affichage, pilotés par lesdits moyens d'élaboration de signaux.

7. Installation selon la revendication 6, caractérisée en ce que ladite mémoire étant du type à lecture cyclique comportant des moyens d'adressage autonome (20) commandant la lecture de chaque image et comportant une entrée de pilotage (30) prévue pour recevoir l'adresse d'un pixel de référence de ladite image, pour initialiser ladite lecture, caractérisé en ce que, ladite table étant mobile, lesdits moyens d'élaboration de signaux représentatifs desdits déplacements relatifs comportent une partie commune avec des moyens de pilotage des déplacements de ladite table (12) ainsi que des moyens de calcul (28) d'une adresse de référence de ladite mémoire correspondant audit pixel de référence, lesdits moyens de calcul recevant en tant que données des signaux prélevés dans lesdits moyens de pilotage et représentatifs desdits déplacements relatifs.

8. Installation selon la revendication 6, dans laquelle ledit moyen d'affichage est un récepteur de télévision, caractérisée en ce que lesdits moyens d'élaboration de signaux représentatifs desdits déplacements relatifs (21, 28a) sont connectés aux moyens de déflexion du tube cathodique dudit récepteur de télévision.

9. Installation selon la revendication 7 ou 8, caractérisée en ce que lesdits signaux représentatifs desdits déplacements relatifs sont prélevés sur des moyens de consigne (21) de moyens d'asservissement des moteurs (25, 26) de déplacement de ladite table (12).

10. Installation selon la revendication 7 ou 8, caractérisée en ce que lesdits signaux représentatifs desdits déplacements relatifs sont prélevés sur des capteurs de position (23a, 24a) associés respectivement aux moteurs (25, 26) de déplacement de la table (12).

**Patentansprüche**

1. Verfahren zur kontrollierten Einstellung der Position eines Patienten in bezug auf eine Röntgeneinrichtung, welche einen Patiententisch (12), eine Meßeinrichtung mit einer Röntgenstrahlquelle (11) und einem Empfänger (13) und Mitteln zur kontrollierten Einstellung der relativen Verschiebung zwischen der Meßeinrichtung und dem Tisch aufweist, und ein Sichtgerät mit einem Bildspeicher (17) zur Aufnahme digitaler, für ein Röntgenbild repräsentativer, Informationen aus dem Empfänger und Anzeigemittel (14) für dieses Bild umfaßt, dadurch gekennzeichnet, daß das Verfahren in der Erfassung und Speicherung eines Bildes durch kurzes Einschalten des Sichtgeräts, in der Wiedergabe des Bildes in einem bestimmten Rahmen oder Perimeter des Anzeigemittels (14), und in der Herbeiführung einer relativen Versetzung (21, 28, 20) besteht, welche repräsentativ für die genannten relativen Verschiebungen zwischen dem Rahmen oder Perimeter und dem Bild ist.

2. Verfahren nach Anspruch 1, bei welchem in an sich bekannter Weise der Speicher zyklisch zur Gewinnung von Signalen gelesen wird, die an eine Fernsehkathodenstrahlröhre geleitet werden, welche das genannte Bild anzeigt, dadurch gekennzeichnet, daß die Adressiermittel (20) des Speichers (17) korrelativ zu den relativen Verschiebungen gesteuert werden.

3. Verfahren nach Anspruch 2, dadurch gekennzeichnet, daß Versetzungssignale erzeugt werden, die repräsentativ für die relativen Verschiebungen sind und in digitale Informationen zur Steuerung (28) der Adressiermittel umgewandelt werden.

4. Verfahren nach Anspruch 1, bei welchem in an sich bekannter Reise der Speicher (17) zyklisch zur Gewinnung von Signalen gelesen wird, welche an eine Fernsehkathodenstrahlröhre zur Wiedergabe des genannten Bildes geleitet werden, dadurch gekennzeichnet, daß Versetzungssignale (28a) erzeugt werden, die repräsentativ für die relativen Verschiebungen sind und an Ablenkmittel der genannten Kathodenstrahlröhre geleitet werden.

5. Verfahren nach einem der vorhergehenden Ansprüche, dadurch gekennzeichnet, daß ein weiteres Röntgenbild jedesmal dann durch kurzes Einschalten des Sichtgeräts gewonnen wird, wenn die Versetzung zu groß erscheint, daß die dem neuen nicht versetzten Bild entsprechenden digitalen Informationen wieder in den Bildspeicher (17) eingeschrieben werden, und daß die relativen Verschiebungen fortgesetzt werden, womit eine neue relative Versetzung wie zitiert eingeleitet wird.

6. Röntgenanlage, die einen Patiententisch (12), eine Meßeinrichtung mit einer Röntgenstrahlquelle (11) und einem Empfanger (13), Mittel zur Einstellung der relativen Verschiebung zwischen der Meßeinrichtung und dem Tisch, und ein Sichtgerät mit einem Bildspeicher (17) zur Aufnahme digitaler, für ein Röntgenbild repräsentativer, Informationen aus dem Empfänger, sowie Anzeigemittel (14) für dieses Bild umfaßt, dadurch gekennzeichnet, daß sie weiter Mittel (21) zur Erzeugung von Signalen besitzt, die repräsentativ für die relativen Verschiebungen sind, und Mittel zur relativen Versetzung des Bildes in bezug auf die Anzeigemittel, die von den Signalerzeugungsmitteln gesteuert werden.

7. Anlage nach Anspruch 6, dadurch gekennzeichnet, daß der Speicher zyklisch gelesen wird, daß autonome Adressiermittel (20), welche das Lesen jedes Bildes steuern, und ein Steuereingang (30) zum Empfang der Adresse eines Referenzbildelements vorgesehen ist, um den Beginn des Lesens einzuleiten, daß der Tisch beweglich ist und die Mittel zur Erzeugung der für die relativen Verschiebungen repräsentativen Signale einen mit den Steuermitteln für die Verschiebung des Tisches (12) gemeinsamen Teil besitzen, sowie Rechenmittel (28) für eine dem Referenzbildelement entsprechende Referenzadresse des Speichers, wobei die Rechenmittel als Daten Signale empfangen, die den Steuermitteln entnommen und repräsentativ für die relativen Verschiebungen sind.

8. Anlage nach Anspruch 6, bei der das Anzeigemittel ein Fernsehempfänger ist, dadurch gekennzeichnet, daß die Mittel zur Erzeugung repräsentativer Signale für die relativen Versetzungen (21, 28a) mit den Ablenkmitteln der Kathodenstrahlröhre des Fernsehempfängers verbunden sind.

9. Anlage nach den Ansprüchen 7 oder 8, dadurch gekennzeichnet, daß die relativen Versetzungen den Sollwert-Einstellungsmitteln (12) des Reglers der Motoren (25, 26) zur Verstellung des Tisches (12) entnommen werden.

10. Anlage nach den Ansprüchen 7 oder 8, dadurch gekennzeichnet, daß die für die relativen Verschiebungen repräsentativen Signale Lagesensoren (23a, 24a) entnommen werden, die jeweils mit den Verstellmotoren (25, 26) des Tisches assoziiert sind.

**Claims**

1. A method for controlling the position of a patient with respect to an X-ray installation, comprising a patient table (12), a measuring assembly comprising an X-ray source (11) and a receiver and means for controlling the relative displacements between the measuring assembly and the table and a visualization system including an image store (17) receiving from said receiver digital information representative for an X-ray image, and a display means (14) for this image, characterized in that an image is taken and stored by briefly actuating the visualization system, by reproducing the image within a determined frame or perimeter of the display means (14) and by causing a relative dislocation (21, 28, 20), which is representative for said relative displacement, between the frame or perimeter and the image.

2. A method according to claim 1, in which, as per se known in the art the store is cyclically read for generating the signals applied to a television cathode ray tube, whereon the image is displayed, characterized in that the addressing means (20) of the store (17) are controlled correlatively with the relative displacements.

3. A method according to claim 2, characterized in that dislocation signals representative for the relative displacements are generated and transformed into digital information for controlling (28) the addressing means.

4. A method according to claim 1, in which, as per se known in the art, the memory is cyclically read for generating the signals applied to a television cathode ray tube, whereon the image is displayed, characterized in that dislocation signals (28a) are generated, which are representative for the relative displacements and applied to the deflection means of the cathode ray tube.

5. A method according to any one of the preceding claims, characterized in that another X-ray image is taken by briefly actuating the visualization system each time the dislocation is judged to be too important, that the digital information corresponding to that new and not dislocated image is re-inscribed in the image store (17) and that the relative displacements are continued, causing again a new relative dislocation as mentioned above.

6. An X-ray installation comprising a patient table (12), a measuring assembly comprising an X-ray source (11) and a receiver and means for controlling the relative displacements between the measuring assembly and the table, and a visualization system including an image store (17) receiving digital information from said receiver representative for an X-ray image, and a display means (14) for this image, characterized in that it further comprises means (21) for generating signals representative for the relative displacements and means for the relative dislocation of the image in relation to said display means, the dislocation means being controlled by said signal generating means.

7. An installation according to claim 6, characterized in that, said store being cyclically read out and comprising autonomous addressing means (20) controlling the read-out of each image and including a pilot input (30) adapted to receive the address of a reference pixel of said image for enabling said read-out operation, characterized in that said table being movable, said means for generating signals representative for relative displacements comprise a common part with means for controlling the table (12) displacements and means (28) for calculating a reference address of said store corresponding to said reference pixel, said calculating means receiving data form signals derived from said control means and representative of said relative displacements.

8. An installation according to claim 6, in which the display means is a television set, characterized in that the means for generating representative signals of the relative dislocations (21, 28a) are connected to the cathode ray tube deflection means of the television set.

9. An installation according to claim 7 or 8, characterized in that said representative signals of said relative dislocations are derived from the control means (21) of the servo means of the motors (25, 26) for the displacement of the table (12).

10. An installation according to claim 7 or 8, characterized in that the representative signals of said relative dislocations are derived from position sensors (23a, 24a) associated respectively with the motors (25, 26) for the displacement of the table (12).

# FIG_1

# FIG_2

1

# FIG_3